# EUROPÄISCHE PATENTANMELDUNG

(11) **EP 3 366 488 A1**
(43) Veröffentlichungstag der Anmeldung: **29.08.2018**
(21) Anmeldenummer: 18157751.1
(22) Anmeldetag: 21.02.2018
(51) Int. Cl.: B43M 11/06, A45D 33/00, A45D 33/02, A45D 34/04, A45D 33/18, A45D 34/00, A45D 40/00, A45D 40/26

(54) **DEO-ROLLER MIT DEKORATIVEM EFFEKT**

(30) Priorität: 24.02.2017 DE 102017001789
(71) Anmelder: Herzmann, Corinna, 47051 Duisburg (DE)
(72) Erfinder: Herzmann, Corinna, 47051 Duisburg (DE)
(74) Vertreter: Demski, Siegfried

(57) **Zusammenfassung**

Die Erfindung betrifft ein Aufsatzelement 2 für einen Behälter 10 zum Auftragen einer Substanz, insbesondere Flüssigkeiten, fließfähige Formulierungen, Pasten oder Pulver, umfassend zumindest einen in einer Lagerung frei beweglichen rotationsymmetrischen Körper 4 zum Auftragen der Substanz, insbesondere auf eine Hautoberfläche. Das Aufsatzelement 2 wird hierbei mit einem Behälter 10 verbunden, in dem die aufzutragende Substanz enthalten ist und mithilfe des Körpers 4, an dem die Substanz bei einer Drehbewegung anhaftet nach außen transportiert, um diese auf beispielsweise einer Haut aufzutragen. Zur Verwendung für Werbemaßnahme oder Erhöhung der Attraktivität ist vorgesehen, dass der Körper 4 einen verschließbaren Hohlraum 5 aufweist und aus zumindest teilweise durchsichtigem Material besteht, wobei der Körper 4 mit einer zumindest teilweise transparenten Flüssigkeit und/oder Festkörperteilchen 6 wenigstens teilweise gefüllt ist. Im Falle einer Rotation wird die Flüssigkeit in dem Körper 4 oder die Festkörperteilchen 6 durcheinander gewirbelt, sodass ein dekoratives Erscheinungsbild entsteht, was sich beispielsweise hervorragend als Werbeträger eignet.

## Beschreibung

Die Erfindung betrifft ein Aufsatzelement für einen Behälter zum Auftragen einer Substanz, insbesondere für Flüssigkeiten, fließfähige Formulierungen, Pasten oder Pulver, umfassend zumindest einen in einer Lagerung frei beweglichen rotationsymmetrischen Körper zum Auftragen der Substanz, insbesondere auf eine Hautoberfläche.

Gattungsgemäße Aufsatzelemente werden beispielsweise als Deo-Roller (Roll-on Stift) angeboten, wobei diese zum Aufsetzen auf einen Behälter, vorzugsweise einen runden Behälter mit einer viskosen Flüssigkeit vorgesehen sind. In der Regel wird das Aufsatzelement auf dem Behälter aufgeschraubt, sodass gleichzeitig eine Abdichtung des Behälters erfolgen kann. Das Aufsatzelement besitzt einen rotationssymmetrischen Körper, der in einer Lagerung frei beweglich aufgenommen ist. Die Lagerung besteht in der Regel aus zwei Halbschalen, welche nach der Aufnahme des rotationssymmetrischen Körpers miteinander verpresst werden. Hierdurch wird der Körper sicher in der Lagerung gehalten, wobei dieser sich frei in jede Richtung drehen kann und zudem über einen entsprechend ausgewählten Durchmesser ein geringfügiger Spalt zwischen der Lagerung und dem Körper vorliegt, sodass bei einer Drehbewegung des Körpers, welcher im weiteren mit der Substanz in Kontakt steht, diese an der Oberfläche anhaften kann und in Folge der Rollbewegung des Körpers nach außen gelangt.

Die Lagerung umschließt hierbei den Körper zu mehr als 50 %, sodass dieser aus der Lagerung nicht nach oben herausfallen kann. Weniger als 50 % der Oberfläche des Körpers ragen aus der Lagerung heraus und sind damit frei zugänglich, sodass über den vorhandenen Spalt zwischen dem Körper und der Lagerung die von dem Körper aufgenommene Substanz, welche an dem Körper anhaftet, bei einer Drehbewegung nach außen transportiert wird. Zu diesem Zweck kann der Körper zusätzlich mit Vertiefungen versehen sein, worin die Substanz bei einer Drehbewegung aus dem Behälter eingelagert wird. Bei Nichtbenutzung des Aufsatzelementes kann dieses mit einer Kappe verschlossen werden, sodass die Substanz nicht verdunstet oder zu Verunreinigung in einer Handtasche etc. führt.

Die bekannten Aufsatzelemente unterscheiden sich hierbei nur geringfügig und bieten lediglich die Möglichkeiten eine Substanz aufzutragen.

Der vorliegenden Erfindung liegt die Aufgabe zugrunde, ein Aufsatzelement bereit zu stellen, welches einen optisch ansprechenden Eindruck vermittelt und darüber hinaus als Werbeträger geeignet ist.

Erfindungsgemäß ist zur Lösung der Aufgabe vorgesehen, dass der Körper einen verschließbaren Hohlraum aufweist und aus zumindest teilweise durchsichtigem Material besteht, wobei der Körper mit einer zumindest teilweise transparenten Flüssigkeit und/oder Festkörperteilchen wenigstens teilweise gefüllt ist. Weitere vorteilhafte Ausgestaltungen der Erfindung ergeben sich aus den Unteransprüchen.

Soweit der rotationssymmetrische Körper dazu verwendet wird, die im Behälter befindliche Substanz auf eine Hautoberfläche zu übertragen, handelt es sich um ein bekanntes Prinzip. Neu ist, dass in dem Körper eine zumindest teilweise transparente Flüssigkeit und/oder Festkörperteilchen enthalten sind, wobei der Körper selbst aus einem durchsichtigen Material besteht und auch nur teilweise gefüllt sein kann. Eine derartige Befüllung kann beispielsweise erfolgen, wie bei Schneekugeln, die als Dekorationsgegenstände angeboten werden. Aufsatzelemente als Roll-On Stifte besitzen hingegen eine praktische Funktion und werden sehr häufig verwendet. Mithilfe der transparenten Flüssigkeit und/oder den Festkörperteilchen wird der Effekt einer Schneekugel nachempfunden und durch den vorhandenen praktischen Nutzen das fertige Produkt aufgrund der erzielbaren Lichteffekte einem breiten Kreis von Interessenten zugeführt. Durch die Rotation und Neigung des Körpers bei der Anwendung wird die Flüssigkeit im Inneren in Bewegung gesetzt und beispielsweise die schneeimitierenden Partikel oder vergleichbare Partikel aufgewirbelt, sodass der bekannte Schneekugeleffekt entsteht. Hierbei kann es sich um eine transparente oder halbtransparente Flüssigkeit handeln, die gegebenenfalls aus mehreren farbigen Flüssigkeiten besteht, die nicht miteinander vermischt werden oder erst durch die rollende Bewegung kurzzeitig miteinander vermischt werden, aber im Ruhezustand zu einer erneuten Separation führen. Darüber hinaus können Festkörperteilchen in der Flüssigkeit enthalten sein, die in dem rotationssymmetrischen Körper herumgewirbelt werden und damit den Schneeeffekt oder vergleichbare Effekte erzeugen. Alternativ können größere figürliche Darstellungen innerhalb des rotationssymmetrischen Körpers eingelagert werden, die aufgrund der Rotation eine Eigenbewegung ausführen, die je nach Flüssigkeit gegebenenfalls verlangsamt erfolgt. Durch die eintretende Bewegung auch nach Abstellen des Roll-On Stiftes bleibt dieser Effekt für eine bestimmte Zeit erhalten und erregt somit die Aufmerksamkeit weiterer Personen. Hierdurch wird ein interessanter optischer und dekorativer Eindruck erweckt, der nicht nur zum Kaufanreiz führt, sondern darüber hinaus einen Blickfang darstellt. Hierbei besteht ohne weiteres die Möglichkeit, dass die Festkörperteilchen selbst fluoreszierend oder lumineszierend sind, sodass dieser Effekt auch bei eintretender Dunkelheit oder in dunklen Räumen kurzfristig wahrgenommen werden kann.

In einer bevorzugten Ausführungsform wird das Aufsatzelement auf einen Behälter aufgeschraubt und besitzt hierzu ein Innengewinde, welches zu dem Außengewinde des Behälters korrespondiert. Das Aufsatzelement besteht in der Regel selbst aus zwei Hauptteilen, und zwar einer Lagerung, die als Aufnahmeschale ausgebildet ist und einem in der Aufnahmeschale frei beweglichen Körper. Die Aufnahmeschale dient zur Halterung des Körpers in dem Aufsatzelement, um beispielsweise ein Herausfallen zu vermeiden. Die Herstellung erfolgt einmal hinsichtlich des rotationssymmetrischen Körpers mit entsprechender Befüllung und andererseits bevorzugt in einem Spritzgießverfahren zur Herstellung der Aufnahmeschalen als Lagerung, die nach Einlegen des Körpers miteinander verbunden werden. Hierbei besteht die Möglichkeit, durch eine Presspassung, Verriegelungsklammern oder aber durch eine Schweißnaht eine dauerhafte Verbindung der beiden Aufnahmeschalen herzustellen. Nachdem das Aufsatzelement hergestellt wurde kann dieses anschließend auf den Behälter aufgeschraubt werden und ist fertig zum Einsatz. Zuvor wird der Behälter mit einer Substanz gefüllt, die mithilfe des Aufsatzelementes auf eine Hautoberfläche übertragen werden kann.

Die Anwendung des Aufsatzelementes ist nicht beschränkt. Das Aufsatzelement kann zum Auftragen jeglicher viskosen Flüssigkeiten, zum Beispiel als Deodorant, Lippenbalsam oder Parfüm zum Einsatz kommen. Denkbar ist ferner die Anwendung des Aufsatzelementes zum Auftragen von viskosen Klebematerialien, zum Beispiel als Roll-On-Klebestift, welcher in jedem Kinderzimmer zu finden ist.

Die Aufnahmeschalen sind bevorzugt ringförmig ausgebildet und bestehen aus Kunststoff. Die Form der Aufnahmeschalen muss an den rotationsymmetrischen Körper angepasst werden, damit sich der rotationsymmetrische Körper in der Schale frei drehen kann.

Je nach Anwendung und Wunsch kann der Körper ein- oder zweiteilig ausgebildet und mit dementsprechend vorhandenen Techniken mit einer transparenten Flüssigkeit und/oder frei beweglichen Festkörperteilchen gefüllt sein.

In einer ersten bevorzugten Ausführungsform besteht der Köper aus einer Kugel, welche zweiteilig ausgebildet ist und mit einer transparenten Flüssigkeit und Partikel gegebenenfalls luftblasenfrei gefüllt ist. Zur Befüllung kann eine handelsübliche Technik je nach Einsatzzweck, Füllungsmaterial und Form des Körpers zum Einsatz kommen.

Durch die Verwendung des Aufsatzelementes, zum Beispiel als Roll-On Applikator eines Deodorants, wird die Kugel in Bewegung gesetzt und die enthaltenen Festkörperteilchen aufgewirbelt, sodass der optische Effekt einer Schneekugel entsteht.

Die frei beweglichen Festkörperteilchen können aus beliebigen Partikeln bestehen, die zu dekorativen Zwecken einsetzbar sind, wie zum Beispiel Perlen, glitzernden und/oder farbigen Sternen, Flocken oder Werbeelementen, welche allesamt durch die rotierende Bewegung herumgewirbelt werden.

Denkbar ist die Herstellung des Köpers in Form einer Kugel, eines Zylinders oder einer Ellipse. Vorgenannte Körper sind zumindest zu einer Achse rotationssymmetrisch gestaltet und können sich in einer Aufnahmeschale mit einer dementsprechenden Form frei bewegen. Eine Kugel besitzt demgegenüber beliebig viele Drehachsen, sodass diese Form bevorzugt zum Einsatz kommt.

Um den optischen Effekt des befüllten Körpers weiterhin zu erhöhen kann das Aufsatzelement in weiterer bevorzugter Ausführungsform einen Hohlraum aufweisen, welcher durch einen oder mehreren Trennwände unterteilt ist. Jeder dieser Räume ist mit einer transparenten Flüssigkeit und den Festkörperteilchen, wie zum Beispiel glitzernden und/oder farbigen Perlen, Sternen, Flocken oder dergleichen gefüllt.

In einer weiteren Ausführungsform der Erfindung können die Räume innerhalb der Kugel mit farbigen Flüssigkeiten gefüllt werden. Somit kann das Aufsatzelement beispielsweise als Werbeartikel oder zur Wiedergabe eines bestimmten Markenzeichens oder einer Flagge verwendet werden. Zu dekorativen oder gewerblichen Zwecken kann der Hohlraum mit einer Struktur ausgestattet sein, welche zum Beispiel ein mit einem Laser graviertes Bild, eine Netzstruktur oder ein beliebig geformtes Zeichen besitzt. Denkbar ist eine Gravierung mit einem Bild, zum Beispiel eines Markenzeichens, einer figürlichen Darstellung, einer Fotografie oder eines Textes, welche sich auf ein bestimmtes Ereignis etc. beziehen.

In einer weiteren bevorzugten Ausführungsform des Aufsatzelementes sind in dem Hohlraum Figuren enthalten. Die Figuren können mit der Wand des Hohlraumes fest verbunden oder im Hohlraum frei beweglich sein.

Um die Haftung der Substanz an der Oberfläche des rotationssymmetrischen Körpers und die Transportfähigkeit gewährleisten zu können, weist der rotationssymmetrischen Köper in einer bevorzugten Ausführungsform eine zumindest teilweise raue Oberfläche auf oder es werden Vertiefungen vorgesehen, in denen die Substanz vorübergehend aufgenommen wird.

Denkbar ist ferner eine Gravierung von Strukturen auf der Oberfläche des rotationssymmetrischen Körpers. Die Strukturen sollen die Transportfähigkeit der Substanz, wobei diese sowohl auf der Außenseite, als auch auf der Innenseite vorgesehen werden können, verbessern. Hierbei kann die Struktur der Oberfläche des rotationssymmetrischen Körper alleine oder in Kombination mit der Struktur auf der inneren Oberfläche des rotationssymmetrischen Körpers ein Muster, ein Bild oder beispielsweise ein Markenzeichen widerspiegeln. Denkbar ist ferner, dass die Struktur auf der Oberfläche des rotationssymmetrischen Körpers mit den Festkörperteilchen und/oder Figuren, welche sich in dem Hohlraum befinden, kombiniert wird. Darüber hinaus kann die Oberfläche zumindest teilweise farbig gestaltet sein.

In einer weiteren bevorzugten Ausführungsform des Aufsatzelementes kann die Strukturierung auf der Oberfläche des rotationssymmetrischen Körpers aus eingravierten Motiven oder geometrischen Formen durch Vertiefungen und Erhöhungen bestehen.

Der besondere Vorteil der vorliegenden Erfindung besteht darin, dass ein herkömmlicher Roll-On-Stift anstelle mit einem üblichen Aufsatzelement mit einem neuartigen Aufsatzelement versehen wird, wodurch eine Reihe von Effekten erzielt werden können. Der Roll-On-Stift besteht hierbei aus einem Behälter in dem die aufzutragende Substanz enthalten ist, welche über den rotationssymmetrischen Körper auf die Haut übertragen werden kann. Zu diesem Zweck weist der Behälter, beispielsweise ein Gewinde auf, sodass das Aufsatzelement mit einem Innengewinde aufgeschraubt werden kann. Das Aufsatzelement besteht im weiteren zumindest aus zwei Aufnahmeschalen, die dauerhaft fest miteinander verbunden werden, sodass der eingeschlossene Körper zwischen den Aufnahmeschalen zwar rotieren kann, aber ansonsten ortsfest gehalten wird. Der Körper selbst wird vorzugsweise aus einem transparenten Material gefertigt und kann befüllt werden. Die Befüllung kann hierbei mit farbigen gelartigen Flüssigkeiten erfolgen, aber ebenso besteht die Möglichkeit, Festkörperteilchen in dem Körper aufzunehmen, die infolge der stattfindenden Rotation des Körpers herumgewirbelt werden und einen Schneekugeleffekt erzeugen. Die verwendeten Farbe oder aber auch die verwendeten Feststoffteilchen können eine bestimmte Symbolik aufweisen und führen somit zu einem interessanten Betrachtungsobjekt, welches beispielsweise auch als Werbemittel eingesetzt werden kann.

Die Erfindung wird im Nachfolgenden anhand der Figuren nochmals erläutert.

Es zeigt
- Fig. 1: eine Seitenansicht eines Roll-On-Stifts mit dem Aufsatzelement,
- Fig. 2: eine Draufsicht des Aufsatzelementes und
- Fig. 3: eine Seitenansicht eine kugelsymmetrischen Körpers

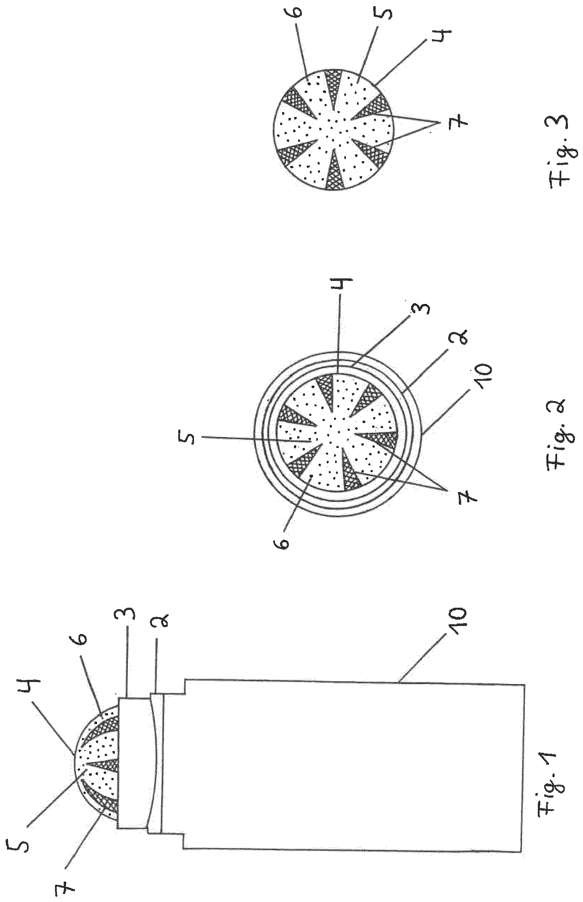

Figur 1 zeigt eine Seitenansicht eines Roll-On-Stiftes 1 mit einem Aufsatzelement 2. Das Aufsatzelement 2 ist zum Auftragen von Substanzen, beispielsweise viskosen Flüssigkeiten auf die Haut gedacht und besteht aus zwei Aufnahmeschalen 3 und einem in den Aufnahmeschalen 3 frei beweglicher Körper 4. Das Aufsatzelement 2 ist zum Aufschrauben auf einem Behälter 10 vorgesehen, in dem sich die aufzutragende Substanz befindet. Im gezeigten Ausführungsbeispiel besteht der bewegliche Körper 4 aus einer frei rotierbaren Kugel, von der weniger als 50 % nach oben aus dem Aufsatzelement 2 herausragt. Im unteren Bereich wird die Kugel nahezu vollständig von dem Aufsatzelement 2 umschlossen, sodass diese weder nach oben noch nach unten herausfallen kann. Die Kugel steht hierbei in Kontakt mit der Substanz, die sich in dem Behälter 10 befindet, welche auf der Kugeloberfläche anhaftet und aufgrund der Rollbewegung nach außen transportiert wird und beispielsweise auf die Haut übertragen werden kann.

Die Aufnahmeschalen 3 sind hierbei fester Bestandteil des Aufsatzelementes 2 und können einstückig mit dem erforderlichen Innengewinde ausgestattet sein oder gegebenenfalls kann nach Zusammenfügen der beiden Aufnahmeschalen 3 der erforderliche Gewindekranz entstehen. Alternativ besteht selbstverständlich die Möglichkeit, dass anstelle eines Gewindes eine andere Verbindung mit dem Behälter 10 erfolgt. Beispielsweise könnte der untere Bereich der Aufnahmeschalen 3 mit dem Behälter 10 verklebt oder verschweißt, insbesondere durch eine Laserschweißnaht verbunden werden. Zum Einsetzten des rotationssymmetrischen Körpers 4, insbesondere der gezeigten Kugel können die beiden Aufnahmeschalen 3 nach dem Einlegen der Kugel miteinander verpresst, verklebt oder ebenfalls verschweißt werden. Anstelle einer Kugel wäre es ebenso denkbar, dass eine zylinderförmige Walze oder Ellipse für den Roll-On-Stift 1 verwendet wird.

Entsprechend der vorliegenden Erfindung kann der kugelsymmetrische Körper 4 einteilig oder beispielsweise zumindest zweiteilig ausgebildet sein. Soweit der Körper 4 hohl ausgebildet ist, kann der Hohlraum mit bekannten Techniken mit einer zumindest teilweise transparenten Flüssigkeit befüllt werden. Hierbei kann es sich um Flüssigkeiten handeln, die farblich gestaltet sind und nicht miteinander vermischen oder nach einer erfolgten Vermischung nach einer gewissen Zeit den ursprünglichen Zustand wieder einnehmen. Des weiteren besteht die Möglichkeit, dass der Hohlraum der Kugel mit Festkörperteilchen 6 gefüllt wird, beispielsweise in Form von Schneeflocken, die aufgrund der Rotation der Kugel durcheinander gewirbelt werden und somit den Effekt, beispielsweise einer Schneekugel herbeiführen. Anstelle von Flocken, insbesondere von glitzernden Flocken oder lumineszierenden Flocken können die Partikel in jeglicher Form zum Einsatz kommen, die einem dekorativen Zweck dienen oder zum Beispiel als Werbeträger verwendet werden.

Sobald der Roll-On-Stift 1 verwendet wird und damit der Körper 4 in eine Rotation versetzt wird, werden die Festkörperteilchen und vorhandenen Flüssigkeiten verwirbelt, sodass ein ansprechender und für jedermann deutlich erkennbarer Effekt entsteht, der als Werbeträger sehr gut genutzt werden kann.

Darüber hinaus kann der vorhandene Hohlraum in dem Körper durch mehrere Trennwände 7 unterteilt sein, sodass verschiedenfarbige Flüssigkeiten oder auch verschiedenfarbige Dekorelemente zum Einsatz kommen können. Ebenso besteht die Möglichkeit, dass ein Teil der Räume mit Flüssigkeiten und ein anderer Teil mit Festkörperteilchen befüllt ist.

Der Roll-On-Stift 1 kann somit zu dekorative oder gewerblichen Zwecken eingesetzt werden, beispielsweise als Werbeartikel oder zur Wiedergabe eines bestimmten Markenzeichens, gegebenenfalls einer Flagge oder kann einen Schriftzug enthalten, der auf ein besonderes Ereignis hinweist. Die einzubringenden festen Festkörperteilchen können hierbei jede beliebige Struktur annehmen. Beispielsweise besteht auch die Möglichkeit, ein mit Laser graviertes Bild innerhalb der Kugel zu erzeugen, welches bei der Benutzung rotiert. Ebenso besteht die Möglichkeit, ein bestimmtes Muster, beispielsweise eine Netzstruktur oder dergleichen zu verwenden, welches ebenfalls durch die Rotation zu einem eindrucksvollen Beobachtungsobjekt führt.

Um den Transport der Substanz zu verbessern, kann der Körper 4 mit einer rauen Oberfläche oder Vertiefungen versehen sein, in denen sich die Substanz ansammelt und somit in erhöhtem Maße auf die Haut übertragen werden kann. Hierbei besteht ohne weiteres die Möglichkeit, dass auch auf der Oberfläche des Körpers 4 ein dreidimensionales Bild oder eine gewünschte Struktur eingraviert wird, welche einerseits den Werbeeffekt und andererseits den Transport der Substanzen verbessert.

Figur 2 zeigt in einer Draufsicht den Roll-On-Stift 1, wobei man von dem Behälter 10 nur den äußeren Rand erkennen kann und zentrisch auf diesem der Körper 4 sowie die Lagerung, bestehend aus den beiden Aufnahmeschalen 3, aufgeschraubt ist.

Figur 3 zeigt in einer Einzelansicht den Körper 4, in Form einer Kugel, deren Oberfläche mit Trennwänden 7 unterteilt ist, sodass verschiedene Flüssigkeiten in die Kugel eingebettet werden können, soweit es sich um geschlossene Trennwände 7 handelt oder die Trennwände 7 zu einer Verstärkung der Wirbelung führen.

### Bezugszeichenliste:

- 1: Roll-On-Stift
- 2: Aufsatzelement
- 3: Aufnahmeschale
- 4: Körper
- 5: Hohlraum
- 6: Festkörperteilchen
- 7: Trennwand
- 10: Behälter

## Patentansprüche

1. Aufsatzelement (2) für einen Behälter 10 zum Auftragen einer Substanz, insbesondere Flüssigkeiten, fließfähige Formulierungen, Pasten oder Pulver, umfassend zumindest einen in einer Lagerung frei beweglichen rotationsymmetrischen Körper (4) zum Auftragen der Substanz, insbesondere auf eine Hautoberfläche,
**dadurch gekennzeichnet,**
**dass** der Körper (4) einen verschließbaren Hohlraum (5) aufweist und aus zumindest teilweise durchsichtigem Material besteht, wobei der Körper (4) mit einer zumindest teilweise transparenten Flüssigkeit und/oder Festkörperteilchen (6) wenigstens teilweise gefüllt ist.

2. Aufsatzelement (2) nach Anspruch 1,
**dadurch gekennzeichnet,**
**dass** der Körper (4) kugel-, zylinder- oder ellipsenförmig ausgebildet ist.

3. Aufsatzelement (2) nach Anspruch 1 oder 2,
**dadurch gekennzeichnet,**
**dass** der Hohlraum (5) durch zumindest eine Trennwand (7) unterteilt ist.

4. Aufsatzelement (2) nach Anspruch 1, 2 oder 3,
**dadurch gekennzeichnet,**
**dass** die Festkörperteilchen (6) aus kleinen frei beweglichen Partikeln, wie zum Beispiel Glimmer, Perlen, Sternen und farbigen Flocken bestehen.

5. Aufsatzelement (2) nach einem der Ansprüchen 1 bis 4,
**dadurch gekennzeichnet,**
**dass** die Festkörperteilchen (6) aus kleinen Figuren oder Strukturen bestehen.

6. Aufsatzelement (2) nach einem der Ansprüchen 1 bis 5,
**dadurch gekennzeichnet,**
**dass** der Körper (4) aus Glas oder Kunststoff besteht.

7. Aufsatzelement (2) nach einem der Ansprüchen 1 bis 6,
**dadurch gekennzeichnet,**
**dass** der Körper (4) zumindest teilweise eine raue Oberfläche aufweist.

8. Aufsatzelement (2) nach Anspruch 7,
**dadurch gekennzeichnet,**
**dass** die Oberfläche durch eine Strukturierung rau ausgebildet ist.

9. Aufsatzelement (2) nach Anspruch 8,
**dadurch gekennzeichnet,**
**dass** die Strukturierung auf der Oberfläche des Körpers (4) aus eingravierten Motiven, Markierungen, Markenzeichen oder aus Vertiefungen und Erhöhungen besteht.

10. Aufsatzelement (2) nach einem der Ansprüche 1 bis 9,
**dadurch gekennzeichnet,**
**dass** die Oberfläche des Körpers (4) zumindest teilweise farbig gestaltet ist.
